# EUROPEAN PATENT APPLICATION

(11) **EP 1 690 509 A1**
(43) Date of publication of application: **16.08.2006**
(21) Application number: 05028161.7
(22) Date of filing: 22.12.2005
(51) Int. Cl.: A61B 17/86

(54) **A prosthesis implant for endo-osseous implantation**

(30) Priority: 11.02.2005 IT MI20050195
(71) Applicant: Galvan, Mauro, 20070 Cerro al Lambro (Milano) (IT); Dell'Aquila, Luca, 26855 Lodi Vecchio (LODI) (IT)
(72) Inventor: Galvan, Mauro, 20070 Cerro al Lambro (Milano) (IT); Dell'Aquila, Luca, 26855 Lodi Vecchio (LODI) (IT)
(74) Representative: Cicogna, Franco

(57) **Abstract**

A prosthesis implant for endo-osseous implantation and immediate and deferred loading osteointegrating orthopaedia comprises a prosthesis and immediate and deferred loading orthopaedia prosthesis body having an endo-osseous portion (2) including, at the apex thereof, a milling cutter portion (3), axially adjoining a tapping portion (4), ending with a stabilizing threaded portion, the endo-osseous portion being suitable for connection to a prosthesis portion (10) associated with a coupling portion of the surgical drill.

## Description

### BACKGROUND OF THE INVENTION

The present invention relates to a prosthesis implant, specifically designed for endo-osseous implantation.

As is known, in endo-osseous implantation, are at present frequently used endo-osseous screws, which are engaged in the portion, having a recess preliminarily made by a milling cutter therein.

In operation, according to methods at present used in conventional implantation procedures, after having located the implanting situs, the osseous cortical portion is pre-drilled by a rosette or pilot cutter, and then a series of sequential millings are performed to achieve the set depth and diameter.

Upon forming said recess, the prosthesis implant is engaged therein, which prosthesis implant can comprise a mono-phase type, i.e. having the stud pin already arranged therein, or of a two-phase type, i.e. separated from the stud pin, to be applied upon achieving the repairing cortical portion osteogenesis.

Thus, it should be apparent that, by using the prior art methods, it is necessary to operate at following times, by forming, at the start, a drilling operation by using suitable milling cutters.

After having performed the drilling operation, the endo-osseous screw is applied, with a risk of an improper dimensional compatibility with the recess and the endo-osseous screw being applied.

Thus, the above method, in addition to requiring a comparatively long operation type, further requires to perform a lot of operating steps, with consequent possible failures because of an imperfect compatibility between the made milled recess and applied implant.

### SUMMARY OF THE INVENTION

Accordingly, the aim of the present invention is to overcome the above mentioned problem, by providing such a prosthesis implant adapted to provide a perfect mating between the cortical recess and applied implant, while greatly simplifying all the required operating steps.

Within the scope of the above mentioned aim, a main object of the invention is to provide such a prosthesis implant which can be so applied as to reduce to a minimum possible traumas, while greatly reducing the repairing osteogenesis time.

Another object of the present invention is to provide such a prosthesis implant which is very reliable and safe in operation.

Yet another object of the present invention is to provide such a prosthesis implant which can be easily made by using easily available elements and materials and which, moreover, is very competitive from a mere economic standpoint.

According to one aspect of the present invention, the above mentioned aim and objects, as well as yet other objects, which will become more apparent hereinafter, are achieved by a prosthesis implant for endo-osseous implantation, characterized in that said prosthesis implant comprises a prosthesis implant body defining an endo-osseous portion having, at an apex part thereof, a milling cutter portion axially adjoining a tapping portion, said tapping portion ending with a stabilizing threaded portion, said endo-osseous portion being coupled to a prosthesis portion associated with a connection portion for connection to a surgical drill.

### BRIEF DESCRIPTION OF THE DRAWINGS

Further characteristics and advantages of the present invention will become more apparent hereinafter from the following detailed disclosure of a preferred, though not exclusive, embodiment of a prosthesis implant specifically designed for endo-osseous implantation, which is illustrated, by way of an indicative, but not limitative, example in the accompanying drawings, where:

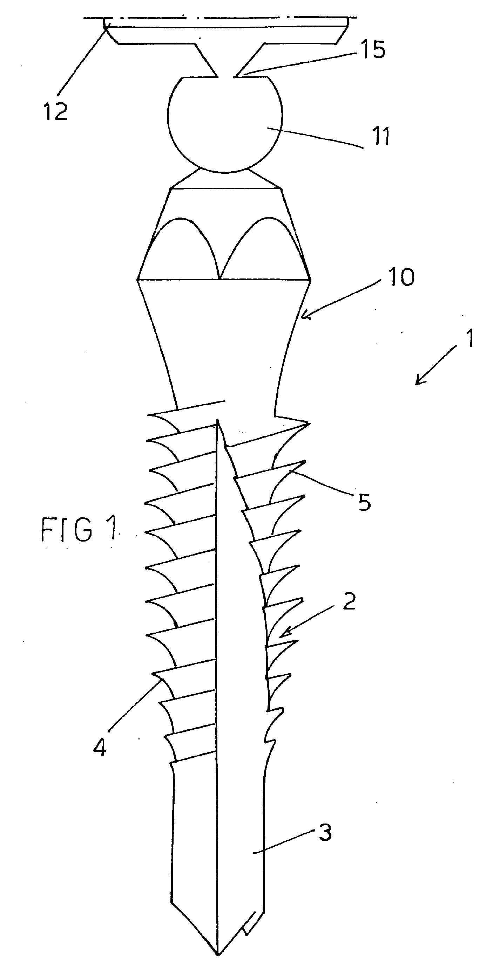

Figure 1 is a schematic view showing the prosthesis implant according to the invention;

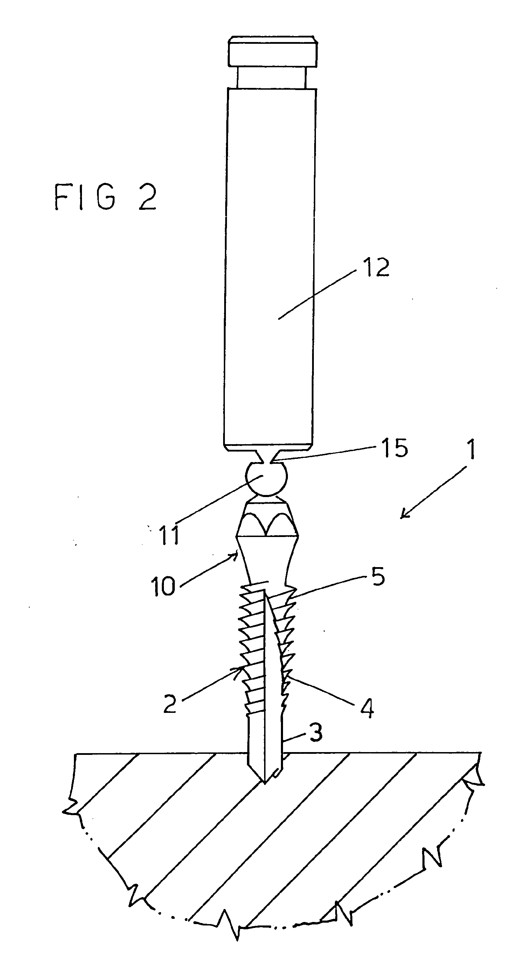

Figure 2 shows a cortical milling operating step;

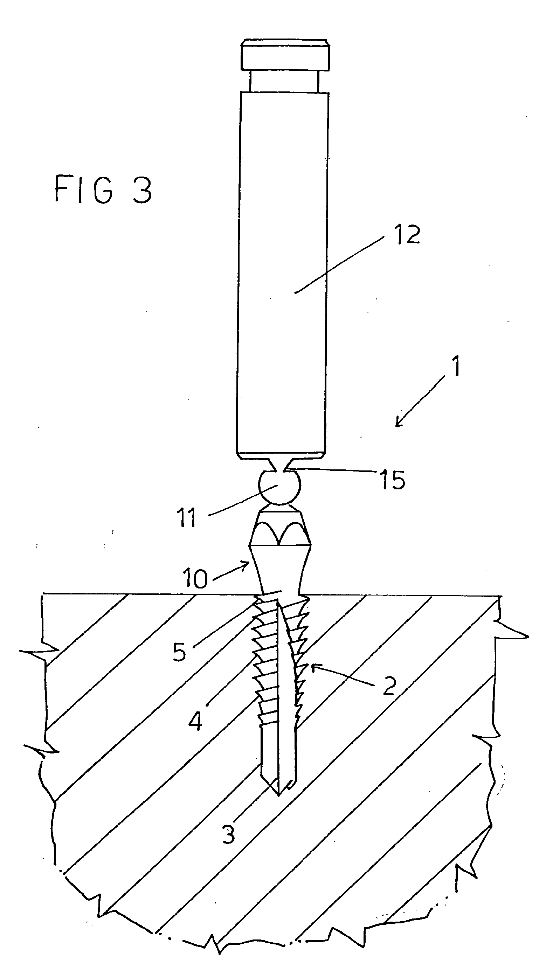

Figure 3 shows the prosthesis implant engaged in the cortical part;
   and

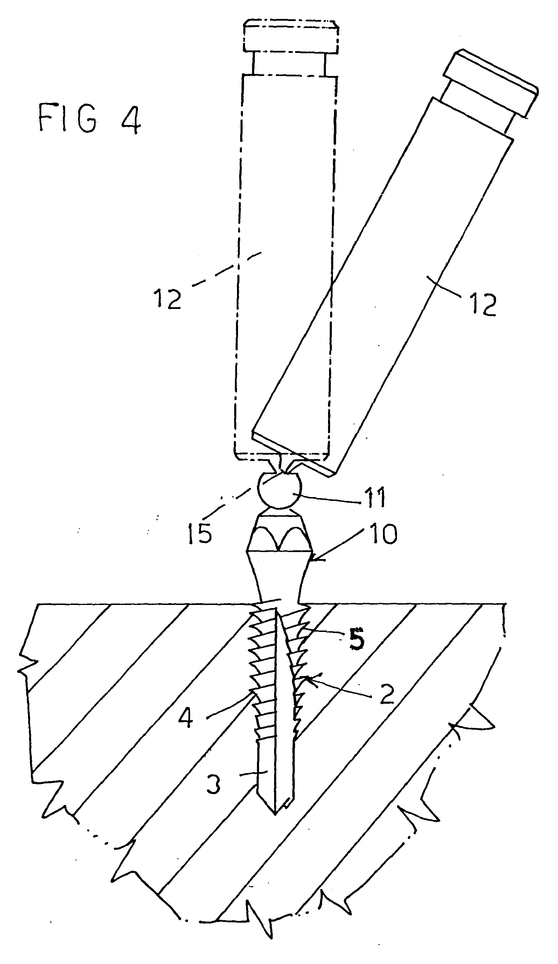

Figure 4 schematically shows an operating step for removing the surgical drill coupling portion.

### DESCRIPTION OF THE PREFERRED EMBODIMENTS

With reference to the number references of the above mentioned figures, the prosthesis implant, specifically designed for endo-osseous implantation, according to the present invention, comprises a prosthesis body, generally indicated by the reference number 1, which is made in accordance with the most macro-and micro surface morphology criteria, to provide an activated surface adapted to improve reparative osteogenesis, upon engaging the implant in the bone.

Said prosthesis body 1 comprises an endo-osseous portion, generally indicated by the reference number 2, which, at an apex or free end portion thereof, comprises a milling cutter portion, which simultaneously operates as a milling tool and an osteo-integrating implant, since it has specifically designed morphologic characteristics, allowing to be easily osteo-integrated.

Said apex portion 2 is coupled to a tapping portion 4, so arranged as to provide a connection between the milling cutter portion 3 and a stabilizing portion 5 which practically comprises a threaded length.

Said threaded length make the endo-osseous part aggressive at the coronal portion thereof, thereby providing the target implantation.

For a single-phase implantation, made as a single-piece with said endo-osseous portion, a prosthesis portion 10 is provided, to define a substantially spherical element 11 for coupling with a connection portion 12, by coupling to the surgical drill.

At the joining region of the coupling portion 11 and coupling element 12, a weakened cross-section has been formed, as generally indicated by the reference number 15, allowing the coupling portion 12 to be easily removed by simply bending it.

In operation, after having assembled the posthesis implant on the surgical drill and actuated said surgical drill at the target rotary speed, the cortical part is started to be drilled, by the above mentioned milling cutter portion.

Thus, the endo-osseous part, upon applying a suitable pressure, will enter the cortical and medullar parts, whereas the apex region, including the milling cutter part, will provide the depth hole.

In particular, the above mentioned tapping part will form, by turning, the recess for the specifically threaded most-coronal part which will be screw engaged in the cortical part, to provide a very aggressive and stabilizing active threaded connection.

Upon ending the rotary movement, the coupling part and endo-osseous portion are broken away from one another, by simply performing a lateral flexure movement.

As stated, the implantation can also comprise a bi-phase type of implantation, the endo-osseous portion of which can be coupled to a stud pin, to be removed by a simple unthreading operation.

From the above disclosure it should be apparent that the invention fully achieves the intended aim and objects.

In particular, the fact is herein stressed that the invention provides a specifically designed prosthesis implantation which, in a single-piece, provides both the milling part for making the recess, and the prosthesis implant.

Thus, the milling cutter will constitute a portion of the prosthesis implant, and the latter will provide an actually suitable assembly, since it is engaged in a recess which is practically provided by the same prosthesis implant.

It should be moreover apparent that the subject miller-implant can be used in an immediate and differed loading osteointegrating orthopaedia application.

The invention, as disclosed, is susceptible to several modifications and variations, all of which will come within the scope of the invention.

Moreover, all the constructional details can be replaced by other technically equivalent elements.

In practicing the invention, the used materials, provided that they are compatible to the intended application, as well as the contingent size and shapes, can be any, depending on requirements.

## Claims

1. A prosthesis implant for endo-osseous implantation, **characterized in that** said prosthesis implant comprises a prosthesis implant body defining an endo-osseous portion having, at an apex part thereof, a milling cutter portion axially adjoining a tapping portion, said tapping portion ending with a stabilizing threaded portion, said endo-osseous portion being coupled to a prosthesis portion associated with a connection portion for connection to a surgical drill.

2. A prosthesis implant, according to claim 1, **characterized in that** said coupling portion is made as a single piece with the prosthesis portion.

3. A prosthesis implant, according to claim 1, **characterized in that** said endo-osseous portion is made as a single-piece with said tapping portion.

4. A prosthesis implant, according to claim 1, **characterized in that** said prosthesis portion is removably coupled to said endo-osseous portion.

5. A prosthesis implant, according to claim 4, **characterized in that** said prosthesis portion defines a substantially spherical connection body, associated with a connection portion for providing a connection with a surgical drill.

6. A prosthesis implant, according to claim 1, **characterized in that** said prosthesis implant comprises, between said coupling element and coupling portion, a reduced cross-section region, to allow said coupling portion to be broken away by a side bending movement.

7. A prosthesis implant, according to claim 1, **characterized in that** said threaded stabilizing portion is aggressively engaged in said cortical part.
